# EUROPEAN PATENT SPECIFICATION

(11) **EP 2 235 705 B1**
(45) Date of publication and mention of the grant of the patent: **24.02.2021**
(21) Application number: 09701047.4
(22) Date of filing: 02.01.2009
(51) Int. Cl.: G08G 1/00, G16H 50/80, G08B 27/00

(54) **METHOD AND SYSTEM FOR WARNING A PARTY PROVIDED WITH A TERMINAL**
VERFAHREN UND SYSTEM ZUR WARNUNG EINES MIT EINEM ENDGERÄT AUSGESTATTETEN TEILNEHMERS
PROCÉDÉ ET SYSTÈME POUR ALERTER UN TIERS DISPOSANT D'UN TERMINAL

(30) Priority: 04.01.2008 FI 20080003
(43) Date of publication of application: 06.10.2010
(73) Proprietor: Medixine OY, 02600 Espoo (FI)
(72) Inventor: JOKINEN, Tapio, FI-02100 Espoo (FI)
(74) Representative: Berggren Oy, Helsinki & Oulu
(86) International application number: PCT/FI2009/050002
(87) International publication number: WO 2009/087269

(56) References cited:
- WO-A1-00/65996
- WO-A1-2006/042900
- WO-A1-2007/057646
- WO-A2-2004/027676
- WO-A2-2005/087091
- JP-A- 2005 063 456
- US-A1- 2004 090 121
- US-A1- 2007 271 298

## Description

### TECHNICAL FIELD OF THE INVENTION

The invention relates to a method and system for warning a party provided with a terminal. In addition the invention relates to a corresponding computer program product.

### BACKGROUND OF THE INVENTION

Different kind of diseases, an outbreak of disease, or expository disease and allergies are inconvenient events. Moreover diseases incur huge financial loss and numbers of day of illness. Many diseases or symptoms are incurred or worsen by environment events, such as weather or changes in weather, like a heat wave, pollen, air impurity, forest fire and its smoke and small particles, as well as dry or hot weather, or other extreme phenomena in weather.

Nowadays weather forecasts are very accurate and weather can be predicted at least roughly for five to ten days or even for longer period in a certain probability. Therefore it is possible to those parties having a certain disease or the like to keep up with the forecast and plan their lives accordingly

US2007271298 A1 relates to the management of asthma by a user taking into account environmental conditions such as weather forecasts as well as location information. WO2005087091 A2 relates to a terminal for displaying medical information to a patient with a chronic, self-managed health condition (e.g. asthma). Environmental conditions can be taken into account in the model and also displayed to the patient based on his location; warnings are provided with a colour scale (green, yellow, red).

### SUMMARY OF THE INVENTION

An object of the invention is to warn parties about a certain environmental event beforehand in order to alleviate or even prevent possible consequences of said event to said party. Especially an object of the invention is to warn the parties individually by avoiding gratuitous warnings, and thus avoiding also frustration and indifference of the parties.

The object of the invention is achieved by the appended independent claims.

According to an embodiment of the invention the party is provided with a mobile terminal, such as a mobile phone, whereupon its location information can be determined based on a cell information in which the mobile phone locates. The cell information can be achieved e.g. from a base station serving the mobile phone at the moment. However, the mobile terminal may also be a laptop computer or other mobile data processing means, which can roam from cell to cell of different base stations, such as in a WLAN data network, and which location can be determined based on the base station serving the mobile terminal on that moment. Location information can be gathered either directly from base stations or asking from the terminal.

However, the terminal may also be a fixed terminal, such as a land lined telephone or desktop computer, which location is fixed and known by the system of the invention.

In order to know when the parties should be warned it is advantageous that each party has an own user profile advantageously in a database of a system, where the user profile comprises information about the party, such as health information or information relating to a medication of the party, age, sex, fitness, and/or size. Health information may be e.g. information about heart disease, allergy, such as pollinosis, asthma, obesity, physical disability, immobility, eye disease, hearing loss, night-blindness, photodermatosis, dry-skinned, renal failure, and/or pregnancy. The gathered accurate profile information of the party ensures that the party is warned only when there is a certain event, which relates at least somehow to the party, and by this way gratuitous warnings can be avoided.

In addition, according to the invention, profile information of the party also comprises information about probable location of the party in a certain moment. This information can be gathered e.g. by following the movements of the party and by analysing location versus time information and especially regularity of the movements of the party. For example it can be detected that the party leaves every weekday a certain location (e.g. home) in the morning at 7:00, moves a certain route and arrives to a certain location (e.g. work) at 7:15, and again in the evening leaves the location at 16:00, moves the same route but reversed direction and arrives the first location (e.g. home) at 16:30. Again it can be detected e.g. that every Wednesday evening the party moves a certain route between 18:00 and 21:00, and every weekend another route starting at 18:00 on Friday and the same route in reversed direction on Sunday at 15:00. Often it is sufficient to determine routes the party uses typically, without any special time information, namely most people typically uses only few routes, whereupon rarely used routes can be ignored.

Location information can also be gathered e.g. by gathering location data from the base stations which serve the mobile terminal, such as a mobile phone, when the party moves with the mobile terminal in the coverage area of different base stations and cells.

According to an embodiment of the invention the party may also input location information to the database of the system manually as such, like home (Helsinki), cottage (Lappeenranta), college (Amsterdam), or location information with time information, such as New York (27.5.2008), Sydney (18.7.2008), and Tokyo (20.10.2008 - 25.10.2008), or routes used typically on weekdays and/or weekends, for example.

Based on gathered data or input data as illustrated above the location where the party will be located in near future, or where (s)he is located typically, can be predicted with a certain probability. Of course the present location can be known very accurately, because it can be ensured e.g. by asking from a network of the mobile terminal (such as from base stations) or asking it directly from the terminal.

According to an embodiment environment information may comprise information relating to weather or weather forecast, traffic information, natural phenomena and /or certain special information relating a certain location, and especially predictions of certain natural phenomena and /or certain special information relating a certain location. Especially environment information is related to certain geographical locations. Information relating to weather forecast may be e.g. tomorrow hot wave, day after tomorrow very hot and dry, a week hence pollen, or tomorrow freezing rain, icy and windy. Traffic information may be information relating to traffic jams or accidents with location information, for example. Natural phenomena information may be information relating to a forest fire and combustion gas, for example.

Now, according to the invention the party being in a certain location and suffering e.g. a hay fever can be warned for example when the weather forecast predicts increasing pollen count in that area. According to another embodiment of the invention the location of the party can be predicted, as depicted above, and now if the weather forecast predicts increasing pollen count in a location within time limit when the party will be at the same location, the party can be warned beforehand so that (s)he can be prepared e.g. with suitable medicines or even keeping away that location on that time.

Furthermore, a warning message also comprises a question for a party, to which the party should also answer. For example, if the party has a renal failure and needs to use an apparatus for dialysis e.g. once a week and it is noticed that e.g. a snowstorm is predicted to a location of the party, (s)he is advantageously warned about the event by a warning message having a question about whether the party is able to leave the place or not before the snowstorm, or does the party have medicines. If the party is not able to leave the place, it may incur some additional tasks, such as informing a corresponding medical staff. This kind of warning is called a dynamic warning, and it can also comprise individualized instructions, such as leaving the place within two days, or get some medicine before the event.

The exemplary embodiments of the invention presented in this document are not to be interpreted to pose limitations to the applicability of the appended claims. The verb "to comprise" is used in this document as an open limitation that does not exclude the existence of also unrecited features. The features recited in depending claims are mutually freely combinable unless otherwise explicitly stated.

In addition, even though it is explained in this document that the warning message is sent to the terminal in order to warn the party, it is only a non limiting example and according to another embodiment of the invention the dynamic warning message sent may also comprise a query to which the user or user's terminal must send an answer. By the query additional information e.g. relating to a situation, such as sufficiency of medicine or state of the user, can be gathered, and more accurate instructions or warning can be produced either to the user or other parties. It should be noted that number of additional questions may be sent to the user's terminal based on the gathered answer and/or other information relating to the environment for example.

For example a query can be sent to the user's terminal and displayed to the user to answer to the query. Based on the user's answer an operation can be performed, such as informing a third party, like a medical staff. Also more detailed instructions or warnings can be sent and displayed to the user via his terminal.

### BRIEF DESCRIPTION OF THE DRAWINGS

Next the invention will be described in greater detail with reference to exemplary embodiments in accordance with the accompanying drawings, in which
Figure 1 illustrates an exemplary warning system according to an advantageous embodiment of the invention, and
Figure 2 illustrates an exemplary warning method according to an advantageous embodiment of the invention.

### DETAILED DESCRIPTION

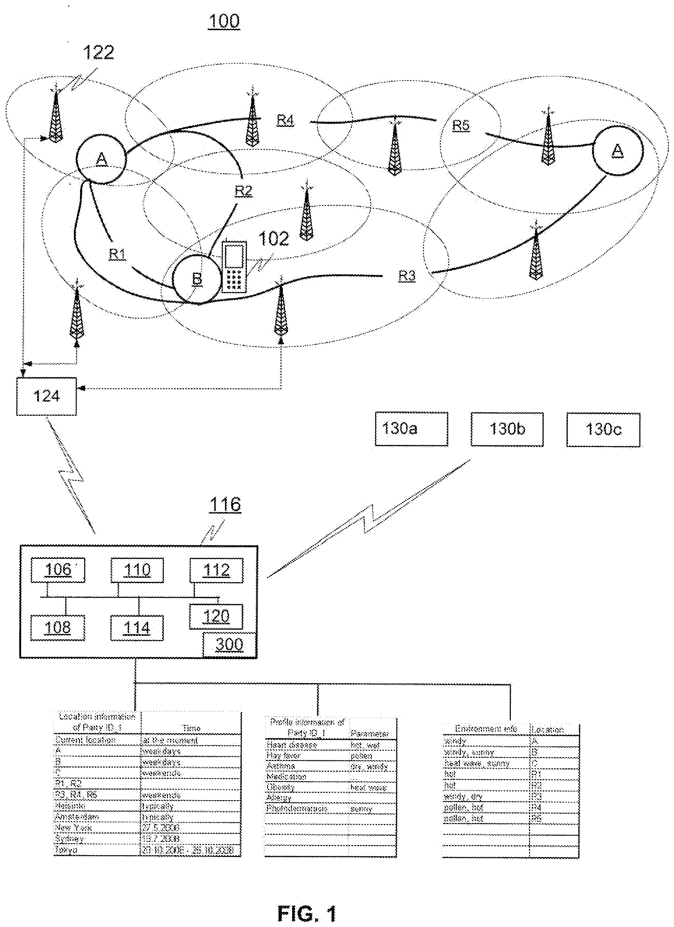

Figure 1 illustrates an exemplary warning system 100 according to an advantageous embodiment of the invention, where a party, who lives in A, is at work in B and travels in weekends to C, is provided with a mobile terminal 102. The system comprises advantageously a database 104a of profile information of the party, a database 104b of location information of the party or terminal, a database 104c of environment information of different geographical locations.

The system comprises also means 106 for checking based on using information of database 104, whether the terminal is located within said geographical location, or even whether said terminal will be located in a certain location in near future with a certain probability or at least typically. In addition the system comprises means 108 for analysing profile information of the party based on information of database 104a and also analysing, whether profile information comprises a parameter which matches with a corresponding parameter of environment information of a geographical location. If there is a matching parameter, the warning system sends a warning message to said terminal using means 110 in order to warn the party.

According to an embodiment the system comprises means 112 of gathering location information of the terminal by enquiring base stations 122 (or other means 124) of the network serving said terminal. In data connection with gathering means are also prediction means 114 for determining most probable routes and locations where the party typically travels or is, or even where the party will be in near future in a certain probability. Furthermore gathering means 112 is typically in a data connection with certain services offering information, such as weather service 130a, traffic service centre 130b, and other service centres 130c offering information for example of natural phenomena and /or certain special information relating a certain location.

Means 106-114 are in an embodiment implemented by a server 116, which can be a computer for example, having a data bus 118 connection means 106-114 to each other in an appropriate way, such as a data processing means 120 for controlling the data flow in the data bus, as well as controlling the functioning of means 106-114. In addition, the databases 104a-c may be implemented outside and independently of the server, but in an advantageous embodiment the databases 104a-c are implemented by the server.

As an example, the party in Figure 1 has a hay fever, as can be seen from his profile information database 104a. In addition, he typically moves between points A and B via routes R1 or R2, mostly via R1. Furthermore he travels also between points A and C, or B and C, mostly between A and B via routes R4 and R4, but sometimes also via route R3. Information relating to these routes and locations are stored in the location information database 104b, as can be seen from Figure 1.

In addition, as an example, the gathering means 112 has gathered weather information from the weather service 130a and stored gathered weather information to the database 104c of environment information with corresponding location data, meaning that for example a heat wave is forecast in location C, and increasing pollen count to locations, which match with the routes R4 and R5. Now, the analysing means 108 is adapted to analyse and compare data above and when predicted location of the party (route R4 and R5) match with location data with forecast increasing pollen count and in addition the party has a hay fever, a warning message is advantageously sent to the party by means 110. The warning message may be for example only a notification about the event, but also more detailed information about duration and seriousness of the event. In addition the warning message may also comprise advisory information (even individualized information), such as proposing another route or suggesting to take appropriate medicaments, in addition the warning message comprises also a question.

The system is advantageously adapted to warn the party for example few days before the event, when the weather service for example (or other environment data service) outputs reliable forecast information about possible weather event in near future.

It should be noted that in an embodiment of the invention the functions of the above mentioned means 106-114 in the server may be implemented at least partly with a computer program product 300 loadable into the internal memory of the computer 116, where said computer program product advantageously comprises software code portions for performing the functions of the above mentioned means. The computer program product 300 may also be stored on a computer usable medium.

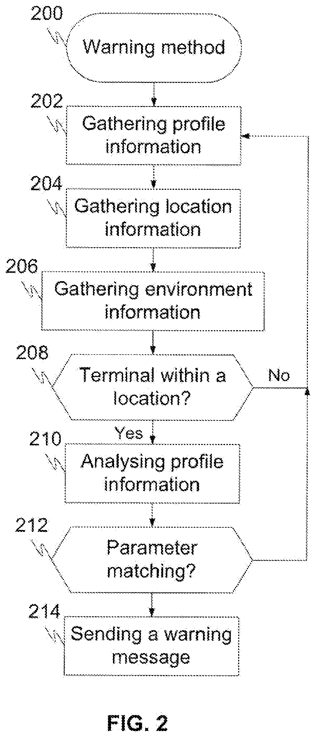

Figure 2 illustrates an exemplary warning method 200 according to an advantageous embodiment of the invention, where at step 202 profile information of the party is gathered, such is disclosed elsewhere in this document. In addition environment information of geographical locations is gathered at step 204, and location information of the terminal (or party) is gathered of step 206. Step 206 may also comprise a sub-step of making predictions about the locations, where the party will be in a certain moment in a certain probability.

The performing of above mentioned steps are not one-time performance, but a continuous, repetitive process.

At step 208 it is checked based on location information of the terminal, whether the terminal is located (or will be located) within the geographical location of at least one event. If it seems that party is located or will be located in that area, party's profile information is analysed at step 210 and determined at step 212, whether profile information comprises a parameter which matches with a corresponding parameter of environment information of said geographical location. If yes, warning message is send to the party's terminal at step 214 in order to warn the party.

If the party is not located or it is not probable that the party will be located in that environment (at step 208) or if none of the parameter in the party's profile information matches with the corresponding parameter of environment information of said geographical location (at step 212), the method is continued for example in step 202 or 208 again.

In connection with the method 200 it should be noted that the order of the steps could also be different than depicted in this example. Especially information can be gathered in different order and continuously. Furthermore in an embodiment data can also be analysed in different order, such as at first checking possible matching with profile information and environment information (step 210 in Figure 2) and after this checkin location information, if there exist any matches (step 208 in Figure 2).

The invention has been explained above with reference to the aforementioned embodiments, and several advantages of the invention have been demonstrated.

## Claims

1. A warning system (100) for warning a party beforehand in a predictive manner. wherein the party is provided with a terminal (102),
the system is provided with profile information of the party, and environment and/or weather information of a geographical location, and location information of the terminal, where said profile information comprises health information and information relating to a medication of the party being conditional on environments and/or weather conditions, and wherein the warning system is **characterized in that** it is adapted to check based on location information of the terminal, whether the terminal is located within said geographical location, and if yes,
the warning system is adapted to analyse profile information of the party and if profile information comprises a parameter of said health information and/or information relating to a medication of the party which matches a corresponding parameter of environment and/or weather information of said geographical location, the warning system is adapted to send a dynamic warning message comprising a question to be answered by the party to said terminal, and based on the answer the warning system is adapted to perform at least one additional task of: informing a third party, providing instructions to be displayed on the terminal, and warning the party via said terminal,
wherein at least the environment and/or weather information of a geographical location is continuously gathered, wherein said profile information of the party comprises information of probable location of the party in a certain moment of time based on gathered location versus time information of the party in the past,
and the system is further adapted to predict the location where the party will be located in the near future with a certain probability based on the gathered data and in addition the system is adapted to use this predicted location information as location information of the terminal when checking whether said terminal is located within said geographical location,
and wherein if the terminal is not located within said geographical region, the dynamic warning message is not sent.

2. The warning system according to claim 1, wherein said terminal is a mobile terminal, such as a mobile phone, and its location information is determined based on a base station serving said terminal, or said terminal is a fixed terminal, such as a land lined telephone.

3. The warning system according to any of preceding claims, wherein profile information of the party comprises information relating to a health status of the party.

4. The warning system according to any of preceding claims, wherein environment information of the geographical location comprises information relating to weather, traffic information, and/or natural phenomena relating a certain location.

5. A warning method (200) for warning a party beforehand in a predictive manner.
wherein the party is provided with a terminal, wherein profile information of the party, environment and/or weather information of a geographical location, and location information of the terminal are gathered (202, 204, 206), and where said profile information comprises health information and information relating to a medication of the party being conditional on environments and/or weather conditions, the warning method being **characterized in that** it comprises checking (208) based on location information of the terminal, whether the terminal is located within said geographical location, and if yes,
analysing (210) profile information of the party and if profile information comprises a parameter of said health information and/or information relating to a medication of the party which matches (212) a corresponding parameter of environment and/or weather information of said geographical location, sending (214) a dynamic warning message comprising a question to be answered by the party to said terminal, and based on the answer performing at least one additional task of: informing third party, providing instructions to be displayed on the terminal, and warning the party via said terminal, ,
wherein the method comprises gathering at least the environment and/or
weather information of a geographical region continuously, gathering said profile information of the party comprises gathering information of probable location of the party in a certain moment of time based on gathered location versus time information of the party in the past, and the method further comprises predicting the location where the party will be located in the near future with a certain probability based on gathered data and in addition using this predicted location information as location information of the terminal when checking whether said terminal is located within said geographical location, and if the terminal is not located within said geographical region, not sending the dynamic warning message.

6. The warning method according to claim 5, wherein said terminal is a mobile terminal, such as a mobile phone, and its location information is determined based on a base station serving said terminal, or said terminal is a fixed terminal, such as a land lined telephone.

7. The warning method according to any of claims 5-6, wherein profile information of the party comprises information relating to a health status of the party.

8. The warning method according to any of claims 5-7, wherein environment information of the geographical location comprises information relating to weather, traffic information, and/or natural phenomena relating a certain location.

9. A computer program product directly loadable into the internal memory of a digital computer, said computer program product comprising software code portions for performing the steps of any of claims 5-8 when said product is run on a computer.

10. A computer program product stored on a computer usable medium comprising computer readable program means for causing a computer to perform the steps of any of claims 5-8 when said product is run on a computer.

## Patentansprüche

1. Warnsystem (100) zum Warnen eines Teilnehmers im Voraus auf prädiktive Weise,
wobei der Teilnehmer mit einem Endgerät (102) versehen ist, dem System Profilinformationen des Teilnehmers und Umgebungs- und/oder Wetterinformationen eines geografischen Standorts und Standortinformationen des Endgeräts bereitgestellt sind, wobei die Profilinformationen Gesundheitsinformationen und Informationen in Bezug auf ein Medikament des Teilnehmers umfassen, das von den Umgebungen und/oder Wetterbedingungen abhängig ist, und wobei das Warnsystem **dadurch gekennzeichnet ist, dass** es angepasst ist, um basierend auf Standortinformationen des Endgeräts zu überprüfen, ob sich das Endgerät innerhalb des geografischen Standorts befindet, und wenn ja, das Warnsystem angepasst ist, um Profilinformationen der Teilnehmers zu analysieren, und wenn Profilinformationen einen Parameter der Gesundheitsinformationen und/oder Informationen in Bezug auf ein Medikament des Teilnehmers umfassen, die mit einem entsprechenden Parameter der Umgebungs- und/oder Wetterinformationen des geografischen Standorts übereinstimmen, wobei das Warnsystem angepasst ist, um eine dynamische Warnmeldung zu senden, die eine Frage enthält, die von dem Teilnehmer an das Endgerät zu beantworten ist, und das Warnsystem angepasst ist, basierend auf der Antwort, um mindestens eine zusätzliche Aufgabe auszuführen: Informieren eines Dritten, Bereitstellen von Anweisungen, die auf dem Endgerät angezeigt werden sollen, und Warnen des Teilnehmers über das Endgerät,
wobei mindestens die Umgebungs- und/oder Wetterinformationen eines geografischen Standorts kontinuierlich gesammelt werden,
wobei die Profilinformationen des Teilnehmers Informationen über den wahrscheinlichen Standort des Teilnehmers in einem bestimmten Zeitpunkt umfassen, basierend auf den gesammelten Standortinformationen gegenüber den Zeitinformationen des Teilnehmers in der Vergangenheit,
und das System ferner angepasst ist, um den Standort, an dem sich der Teilnehmer in naher Zukunft befinden wird, mit einer bestimmten Wahrscheinlichkeit basierend auf den gesammelten Daten vorherzusagen, und das System zusätzlich angepasst ist, um diese vorhergesagten Standortinformationen als Standortinformationen des Endgeräts zu verwenden, während überprüft wird, ob sich das Endgerät innerhalb des geografischen Standorts befindet,
und wobei, wenn sich das Endgerät nicht innerhalb der geografischen Region befindet, die dynamische Warnmeldung nicht gesendet wird.

2. Warnsystem nach Anspruch 1, wobei das Endgerät ein mobiles Endgerät ist, wie beispielsweise ein Mobiltelefon, und seine Standortinformationen basierend auf einer Basisstation bestimmt werden, die das Endgerät bedient, oder das Endgerät ein festes Endgerät ist, wie beispielsweise ein Festnetztelefon.

3. Warnsystem nach einem der vorhergehenden Ansprüche, wobei Profilinformationen des Teilnehmers Informationen umfassen, die sich auf einen Gesundheitszustand des Teilnehmers beziehen.

4. Warnsystem nach einem der vorhergehenden Ansprüche, wobei Umgebungsinformationen des geografischen Standorts Informationen bezüglich des Wetters, Verkehrsinformationen und/oder Naturphänomene umfassen, die einen bestimmten Standort betreffen.

5. Warnverfahren (200) zum Warnen eines Teilnehmers im Voraus auf prädiktive Weise,
wobei der Teilnehmer mit einem Endgerät versehen ist, wobei Profilinformationen des Teilnehmers, Umgebungs- und/oder Wetterinformationen eines geografischen Standorts und Standortinformationen des Endgeräts gesammelt werden (202, 204, 206), und wobei die Profilinformationen Gesundheitsinformationen und Informationen in Bezug auf ein Medikament des Teilnehmers umfassen, das von Umgebungen und/oder Wetterbedingungen abhängig ist, wobei das Warnverfahren **dadurch gekennzeichnet ist, dass** es Folgendes umfasst Überprüfen (208) anhand der Standortinformationen des Endgeräts, ob sich das Endgerät innerhalb des geografischen Standorts befindet, und wenn ja, Analysieren (210) von Profilinformationen des Teilnehmers, und ob Profilinformationen einen Parameter der Gesundheitsinformationen und/oder Informationen in Bezug auf ein Medikament des Teilnehmers umfassen, die einem entsprechenden Parameter der Umwelt- und/oder Wetterinformationen des geografischen Standorts übereinstimmen (212),
Senden (214) einer dynamischen Warnmeldung, die eine Frage umfasst, die von dem Teilnehmer an das Endgerät zu beantworten ist, und basierend auf der Antwort, Ausführen mindestens einer zusätzlichen Aufgabe:
Informieren eines Dritten, Bereitstellen von Anweisungen, die auf dem Endgerät angezeigt werden sollen, und Warnen des Teilnehmers über das Endgerät,
wobei das Verfahren das kontinuierliche Sammeln mindestens der Umgebungs- und/oder Wetterinformationen einer geografischen Region umfasst, das Sammeln der Profilinformationen des Teilnehmers das Sammeln von Informationen über den wahrscheinlichen Standort des Teilnehmers in einem bestimmten Zeitpunkt basierend auf den gesammelten Standort- / Zeitinformationen des Teilnehmers in der Vergangenheit umfasst, und das Verfahren ferner das Vorhersagen des Standorts, an dem sich der Teilnehmer in naher Zukunft mit einer bestimmten Wahrscheinlichkeit auf der Grundlage der gesammelten Daten befinden wird, und zusätzlich das Verwenden der vorhergesagten Standortinformationen als Standortinformationen des Endgeräts umfasst, während überprüft wird, ob sich das Endgerät innerhalb des geografischen Standorts befindet,
und wenn sich das Endgerät nicht in der geografischen Region befindet, die dynamische Warnmeldung nicht gesendet wird.

6. Warnverfahren nach Anspruch 5, wobei das Endgerät ein mobiles Endgerät ist, wie beispielsweise ein Mobiltelefon, und seine Standortinformationen basierend auf einer Basisstation bestimmt werden, die das Endgerät bedient, oder das Endgerät ein festes Endgerät ist, wie beispielsweise ein Festnetztelefon.

7. Warnverfahren nach einem der Ansprüche 5 bis 6, wobei Profilinformationen desTeilnehmers Informationen umfassen, die sich auf einen Gesundheitszustand des Teilnehmers beziehen.

8. Warnverfahren nach einem der Ansprüche 5 bis 7, wobei Umgebungsinformationen des geografischen Standorts Informationen bezüglich des Wetters, Verkehrsinformationen und/oder Naturphänomene umfassen, die einen bestimmten Standort betreffen.

9. Computerprogrammprodukt, das direkt in den internen Speicher eines digitalen Computers geladen werden kann, wobei das Computerprogrammprodukt Softwarecodeabschnitte zum Ausführen der Schritte eines der Ansprüche 5 bis 8 umfasst, wenn das Produkt auf einem Computer ausgeführt wird.

10. Computerprogrammprodukt, das auf einem von Computern verwendbaren Medium gespeichert ist, das ein computerlesbares Programmmittel umfasst, um einen Computer zu veranlassen, die Schritte eines der Ansprüche 5 bis 8 auszuführen, wenn das Produkt auf einem Computer ausgeführt wird.

## Revendications

1. Système d'avertissement (100) destiné à avertir un correspondant à l'avance de manière prédictive,
dans lequel le correspondant dispose d'un terminal (102), le système dispose d'informations de profil du correspondant, d'informations d'environnement et/ou météorologiques d'un emplacement géographique et d'informations d'emplacement du terminal, lesdites informations de profil comprenant des informations de santé et des informations relatives à un médicament du correspondant conditionnées aux environnements et/ou aux conditions météorologiques, et dans lequel le système d'avertissement est **caractérisé en ce qu'**il est
conçu pour vérifier, sur la base des informations d'emplacement du terminal, si le terminal est situé dans ledit emplacement géographique, et si tel est le cas,
le système d'avertissement est conçu pour analyser les informations de profil du correspondant et, si les informations de profil comprennent un paramètre desdites informations de santé et/ou desdites informations relatives à un médicament du correspondant qui correspond à un paramètre correspondant des informations d'environnement et/ou météorologiques dudit emplacement géographique, le système d'avertissement est conçu pour envoyer audit terminal un message d'avertissement dynamique comprenant une question à laquelle le correspondant doit répondre et, selon la réponse, le système d'avertissement est conçu pour exécuter au moins une tâche supplémentaire parmi : informer un tiers, fournir des instructions à afficher sur le terminal et avertir le correspondant par l'intermédiaire dudit terminal,
dans lequel au moins les informations d'environnement et/ou météorologiques d'un emplacement géographique sont collectées en continu,
dans lequel lesdites informations de profil du correspondant comprennent les informations d'un emplacement probable du correspondant à un certain moment sur la base d'informations d'emplacement en fonction du temps recueillies du correspondant dans le passé,
et le système est en outre conçu pour prévoir avec une certaine probabilité l'emplacement où le correspondant sera situé dans un avenir proche sur la base des données collectées et, en outre, le système est conçu pour utiliser ces informations d'emplacement prévues comme informations d'emplacement du terminal lors de la vérification du fait que ledit terminal est situé ou non dans ledit emplacement géographique,
et dans lequel, si le terminal n'est pas situé dans ladite zone géographique, le message d'avertissement dynamique n'est pas envoyé.

2. Système d'avertissement selon la revendication 1, dans lequel ledit terminal est un terminal mobile, tel qu'un téléphone mobile, et ses informations d'emplacement sont déterminées sur la base d'une station de base desservant ledit terminal, ou ledit terminal est un terminal fixe, tel qu'un téléphone de ligne terrestre.

3. Système d'avertissement selon l'une quelconque des revendications précédentes, dans lequel les informations de profil du correspondant comprennent des informations relatives à un état de santé du correspondant.

4. Système d'avertissement selon l'une quelconque des revendications précédentes, dans lequel les informations d'environnement de l'emplacement géographique comprennent des informations relatives aux conditions météorologiques, aux informations sur le trafic et/ou aux phénomènes naturels concernant un certain emplacement.

5. Procédé d'avertissement (200) destiné à avertir un correspondant à l'avance de manière prédictive, dans lequel le correspondant dispose d'un terminal, dans lequel des informations de profil du correspondant, des informations d'environnement et/ou météorologiques d'un emplacement géographique et des informations d'emplacement du terminal sont collectées (202, 204, 206), et lesdites informations de profil comprenant des informations de santé et des informations relatives à un médicament du correspondant conditionnées aux environnements et/ou aux conditions météorologiques, le procédé d'avertissement étant **caractérisé en ce qu'**il comprend
la vérification (208), sur la base des informations d'emplacement du terminal, du fait que le terminal est situé ou non dans ledit emplacement géographique, et si tel est le cas,
l'analyse (210) des informations de profil du correspondant et, si les informations de profil comprennent un paramètre desdites informations de santé et/ou desdites informations relatives à un médicament du correspondant qui correspond (212) à un paramètre correspondant des informations d'environnement et/ou météorologiques dudit emplacement géographique,
l'envoi (214) audit terminal d'un message d'avertissement dynamique comprenant une question à laquelle le correspondant doit répondre et, selon la réponse, l'exécution d'au moins une tâche supplémentaire parmi :
informer un tiers, fournir des instructions à afficher sur le terminal et avertir le correspondant par l'intermédiaire dudit terminal,
dans lequel le procédé comprend la collecte en continu au moins des informations d'environnement et/ou météorologiques d'une zone géographique, la collecte desdites informations de profil du correspondant comprenant la collecte d'informations de l'emplacement probable du correspondant à un certain moment sur la base d'informations d'emplacement en fonction du temps recueillies du correspondant dans le passé, et le procédé comprend en outre la prévision avec une certaine probabilité de l'emplacement où le correspondant sera situé dans un avenir proche sur la base des données collectées et, en outre, l'utilisation de ces informations d'emplacement prévues comme informations d'emplacement du terminal lors de la vérification du fait que ledit terminal est situé ou non dans ledit emplacement géographique, et si le terminal n'est pas situé dans ladite zone géographique, le fait de ne pas envoyer le message d'avertissement dynamique.

6. Procédé d'avertissement selon la revendication 5, dans lequel ledit terminal est un terminal mobile, tel qu'un téléphone mobile, et ses informations d'emplacement sont déterminées sur la base d'une station de base desservant ledit terminal, ou ledit terminal est un terminal fixe, tel qu'un téléphone de ligne terrestre.

7. Procédé d'avertissement selon l'une quelconque des revendications 5 et 6, dans lequel les informations de profil du correspondant comprennent des informations relatives à un état de santé du correspondant.

8. Procédé d'avertissement selon l'une quelconque des revendications 5 à 7, dans lequel les informations d'environnement de l'emplacement géographique comprennent des informations relatives aux conditions météorologiques, aux informations sur le trafic et/ou aux phénomènes naturels concernant un certain emplacement.

9. Produit de programme informatique pouvant être directement chargé dans la mémoire interne d'un ordinateur numérique, ledit produit de programme informatique comprenant des parties de code logiciel pour exécuter les étapes selon l'une quelconque des revendications 5 à 8 lorsque ledit produit est exécuté sur un ordinateur.

10. Produit de programme informatique stocké sur un support utilisable par ordinateur comprenant un moyen de programme lisible par ordinateur destiné à amener un ordinateur à exécuter les étapes selon l'une quelconque des revendications 5 à 8 lorsque ledit produit est exécuté sur un ordinateur.
